# EUROPEAN PATENT SPECIFICATION

(11) **EP 2 393 535 B1**
(45) Date of publication and mention of the grant of the patent: **25.03.2015**
(21) Application number: 10702303.8
(22) Date of filing: 03.02.2010
(51) Int. Cl.: A61M 5/145

(54) **MEDICAMENT DELIVERY DEVICES**
VORRICHTUNGEN ZUR MEDIKAMENTENVERABREICHUNG
DISPOSITIFS D'ADMINISTRATION DE MÉDICAMENT

(30) Priority: 05.02.2009 EP 09001577
(43) Date of publication of application: 14.12.2011
(73) Proprietor: Sanofi-Aventis Deutschland GmbH, 65929 Frankfurt am Main (DE)
(72) Inventor: BRÜGGEMANN, Ulrich, 65926 Frankfurt am Main (DE); JONES, Christopher, Broadway Worcestershire WR12 7AL (GB); DRAPER, Paul, Leamington Spa CV31 2TG (GB)
(86) International application number: PCT/EP2010/051270
(87) International publication number: WO 2010/089308

(56) References cited:
- WO-A-97/00091
- US-A1- 2004 000 818

## Description

This invention relates to medicament delivery devices for delivering medicine to the human or animal body and in particular, but not exclusively, to devices having a replaceable medicament cartridge. Such devices are commonly used by those with diabetes for the administration of insulin.

Medicament delivery devices are routinely used by persons without formal medical training, i.e. patients where self-management of their condition is increasingly common. These circumstances set a number of requirements for medicament delivery devices of this kind. The injector should be robust in construction, yet easy to use in terms of its operation by a user and the manipulation of the parts. In the case of those with diabetes, many users will be of impaired vision and may also be physically infirm. Devices that are too large of cumbersome may therefore prove difficult to use, particularly someone with reduced dexterity.

Patent Specification WO 03/061736 describes a medicament injection device with an axially displaceable lead screw. In this device, the lead screw is in threaded piston engagement with a drive mechanism that comprises a gear and electric motor. The rotational drive of the motor is translated into an axial force by the gear and threaded piston, the axial force being applied to a bung or elastomeric piston of the medicament cartridge. This driving action is operative to expel an amount of the medicine from the cartridge. In order to fully empty the cartridge, the length of the lead screw has to be at least equal to the length of the cartridge, making the total length of the device at least twice the length of the cartridge. A problem with this type of injection device is that the total length is too long to fit unobtrusively within into a jacket pocket or handbag.

In order to produce a device that is shorter than twice the length of the cartridge, a telescopic piston rod has been developed. Such a telescopic piston rod is shown, utilized in an infusion device, in WO 97/00091. This patent specification describes a linear-transmission syringe plunger that has a piston rod comprising a plurality of pieces or parts connected to one another by mating threads. The distal part of the telescopic piston rod is connected to the elastomeric piston of the syringe, and is prevented from rotating by a number of bushings surrounding the telescopic piston rod. The bushing of the largest diameter needs to fit within the diameter of the syringe with the consequence that the telescopic piston is only suitable for a syringe of considerable size.

Another example of prior art medicament delivery devices is given in US 2004/000818 A.

It is an aim of the present invention to provide a medication delivery device that has a telescopic piston rod assembly that is more compact.

According to the present invention, there is provided a medicament delivery device comprising: a housing; a telescopic piston rod assembly for driving a bung of a medicament container; and a drive mechanism for the telescopic piston rod assembly, wherein the telescopic piston rod assembly has an input drive telescopically coupled to a plunger for driving the bung via an intermediate coupling; characterised in that:
the intermediate coupling comprises: first and second members telescopically coupled to one another, the plunger being telescopically coupled to one of the first and second members and the input drive being telescopically coupled to the other one of the first and second members; and a key for restraining rotation of the plunger relative to the housing;
wherein the input drive, the first and the second members, and the plunger, cooperate to expand or retract with respect to one another when the input drive is driven by the drive mechanism.

The key may comprise first and second sleeves that are telescopically coupled and keyed to one another, the plunger and the housing so that they are axially but not rotationally moveable relative to the housing. In other words, the plunger and intermediate coupling are not rotatable relative to the housing. The key may be effected by way of splines and corresponding grooves or recesses provided in the plunger, the first and second sleeves. The first and second members may be generally cylindrical to form first and second cylinders. The first and second sleeves preferably have lengths that substantially correspond to the respective lengths of the first and second cylinders. The plunger and the input drive preferably also have lengths that correspond to the lengths of the first and second cylinders so as to minimise the length of the telescopic piston rod assembly when in a retracted state. The telescopic relationship between the plunger, the first and second cylinders, and the input drive are such that their respective diameters progressively decrease from the plunger to the input drive. Conversely, the telescopic relationship between the plunger, the first and second sleeves is such that their respective diameters progressively increase from the plunger to the input drive.

The plunger may have an internal screw thread that cooperates with an outer screw thread of the second cylinder. The second cylinder is provided with an inner screw thread at its end remote from the plunger that cooperates with an outer screw thread of the first cylinder. Similarly, the first cylinder is provided with an inner screw thread at its end remote from the second cylinder that cooperates with an outer screw thread provided on the input drive. An input gear is fixed to the end of the input drive remote from the first cylinder for engagement with a gear train of the drive mechanism. This provides for transmission of drive from a battery powered motor of the drive mechanism to the telescopic piston rod assembly via the gear train.

Embodiments of the present invention are not limited to telescopic piston rod assemblies that extend and contract in any particular phased or staged manner. Extension/contraction of the telescopic piston rod assembly from/to a retracted state where the plunger, the first and second cylinders and the sleeves lie within one another may, in some embodiments, be performed progressively. This may be in three phases or stages or, in other embodiments, the extension/contraction of the components of the telescopic piston rod may be sequential. In yet further embodiments, the extension or contraction of the telescopic piston rod assembly components may be in no definite predictable order. The order in which the plunger, the first and second members and the input drive extend or contract may differ according to the design of internal and external threads, as well as the relative diameters and frictional characteristics, at interfaces of these components.

In phase 1, the input drive is rotated by the gear train of the drive mechanism engaging with the input gear but is constrained in an axial direction by the housing. The input drive therefore transfers axial forces into the housing while transferring the torque input from the drive mechanism into the first cylinder. During phase 1, the first cylinder, the second cylinder, the first and second sleeves and the plunger move bodily and axially (but without rotation) along the length of the input drive. This occurs on account of: the first cylinder transferring the torque input from the input drive and provides an axial force into the second cylinder; the second cylinder transferring the axial input force from the first cylinder and transferring the axial input force into the plunger; the first sleeve reacts the torque from the second sleeve and transfers it into the housing; the second sleeve reacts the torque from the plunger and transfers it into the first sleeve; and the plunger transfers the axial force into the bung of the medicament cartridge in order to dispense the medicament.

Phase 2 begins when the first cylinder has fully extended, that is, has moved down the length of the input drive until relative rotation between them is arrested by an extension stop. At this point, the first cylinder no longer moves axially but rotates with the input drive. The first cylinder then transfers the input torque from the input drive into the second cylinder, thus causing the second cylinder to progress axially along the first cylinder without rotation. The second cylinder transfers the torque input from the first cylinder and provides an axial force into the plunger. The plunger moves axially without rotation. The plunger transfers the torque input from the second cylinder and provides an axial force into the bung of the medicament cartridge in order to dispense the medicament. During phase 2, the first sleeve does not move but reacts the torque from the second sleeve and transfers it into the drive mechanism housing. The second sleeve does not move and reacts the torque from the plunger and transfers it into the first sleeve so that it does not rotate. The keying of the sleeves may be facilitated by way of light fit between axially running splines and corresponding grooves. The order of movement of the sleeves is not essential or predictable. It will depend on the relative friction between the sleeves and the plunger. The grooves would be blind at one end (not shown) to prevent the sleeves and the plunger decoupling.

Phase 3 begins when the second cylinder has fully extended, that is, has moved down the length of the first cylinder until relative rotation is arrested by a second extension stop, it no longer moves axially but rotates with the input drive and the first cylinder. The second cylinder then transfers the torque from the first cylinder into the plunger so that the plunger moves axially along the length of the second cylinder without rotating. The plunger is keyed to the second sleeve so that it is prevented from rotating with the input drive, thereby achieving relative rotation with the second cylinder. A third extension stop is provided to arrest relative rotation between the plunger and the second cylinder when the plunger is fully extended relative thereto. The first, second and third extension stops, which may be provided by closed end threads, also serve to prevent the telescopic piston rod assembly from de-coupling. Clip-on stop rings may be provided for preventing the screw components from decoupling. The clip-on stop rings have been specifically developed to provide sufficient stop strength without requiring a bonding process or constraining the material selection of the screw components.

The order of operation of the first and second sleeves is governed by the frictional efficiency of the drive screws. The first cylinder will tend to move first in preference to the second cylinder and plunger during the first phase of the sequential extension of the telescopic piston rod assembly due to the relatively low friction between the smallest diameter screw interface between the input drive and the first cylinder. In other words, the successive extension of the first cylinder, then second cylinder, and finally the plunger is enabled by the increasing friction arising as their respective screw interfaces.

The term "medicament delivery device" according to instant invention shall mean a single-dose or multi-dose or pre-set dose or pre-defined, disposable or re-useable device designed to dispense a user selectable or pre-defined dose of a medicinal product, preferably multiple doses, e.g. insulin, growth hormones, low molecular weight heparins, and their analogues and/or derivatives etc. Said device may be of any shape, e.g. compact or pen-type. Dose delivery may be provided through a mechanical (optionally manual) or electrical drive mechanism or stored energy drive mechanism, such as a spring, etc. Dose selection may be provided through a manual mechanism or electronic mechanism. Additionally, said device may contain components designed to monitor physiological properties such as blood glucose levels, etc. Furthermore, the said device may comprise a needle or may be needle-free. In particular, the term "medicament delivery device" may refer to a needle-based device providing multiple doses having an electrical drive mechanism, which is designed for use by persons without formal medical training such as patients. Preferably, the drug delivery device is of the automated-type, i.e. an auto-injector.

The term "housing" according to instant invention shall preferably mean any exterior housing ("main housing", "body", "shell") or interior housing ("insert", "inner body") having a unidirectional axial coupling to prevent proximal movement of specific components. The housing may be designed to enable the safe, correct, and comfortable handling of the drug delivery device or any of its mechanism. Usually, it is designed to house, fix, protect, guide, and/or engage with any of the inner components of the drug delivery device (e.g., the drive mechanism, cartridge, plunger, piston rod) by limiting the exposure to contaminants, such as liquid, dust, dirt etc. In general, the housing may be unitary or a multipart component of tubular or non-tubular shape. Usually, the exterior housing serves to house a cartridge from which a number of doses of a medicinal product may by dispensed.

The term "motor" according to the instant invention shall preferably mean any motorised means for driving the gearing system and ultimately the input drive means. In the instant invention a stepper motor is preferably utilised although any means for driving the gearing system or the drive means, including a mechanical or manual actuation means, may also be incorporated into the device.

The "proximal end" of the device or a component of the device shall mean the end, which is furthest away from the dispensing end of the device.

The "distal end" of the device or a component of the device shall mean the end, which is closest to the dispensing end of the device.

Embodiments of the present invention provide a four-stage telescopic piston rod assembly for more compact medicament delivery devices, both in terms of the axial length of the device as well as the diameter of the medicament container. Telescopic piston rod assemblies embodying the invention may be made from extremely thin walled injection mounded parts, using sophisticated technical plastics materials. Specifically, the input drive has a diameter that has been reduced to provide space for the surrounding components. Consequently, devices embodying the invention may be usefully deployed in re-useable medicament delivery devices that comprise replaceable medicament cartridges and may also be deployed within an auto-injector device.

The invention will now be further described by way of example with reference to the accompanying drawings, in which like reference numerals designate like elements:
Figure 1 is a front view of a medicament delivery device that may include an embodiment of the present invention;
Figure 2 is a front view of the medicament delivery device of Figure 1 with a medicament cartridge door shown in an open position for receiving a medicament cartridge;
Figure 3 is a perspective view of a telescopic piston rod assembly embodying the present invention, showing a motor, gear and drive mechanism housing;
Figure 4 is a sectional perspective view of the motor, the gear train and drive mechanism of Figure 3;
Figure 5 is a sectional view of the telescopic piston rod assembly in an extended position within a medicament cartridge;
Figure 6 is a sectional view of a modified embodiment of the door locking member and the telescopic piston rod assembly in an extended position;
Figure 7 is a sectional view of a modified embodiment of the telescopic piston rod assembly of Figure 3 in a retracted state, and a door locking member in a forward position; and
Figure 8 is a sectional view of a modified embodiment of Figure 7 with the door locking member in a rearward position.

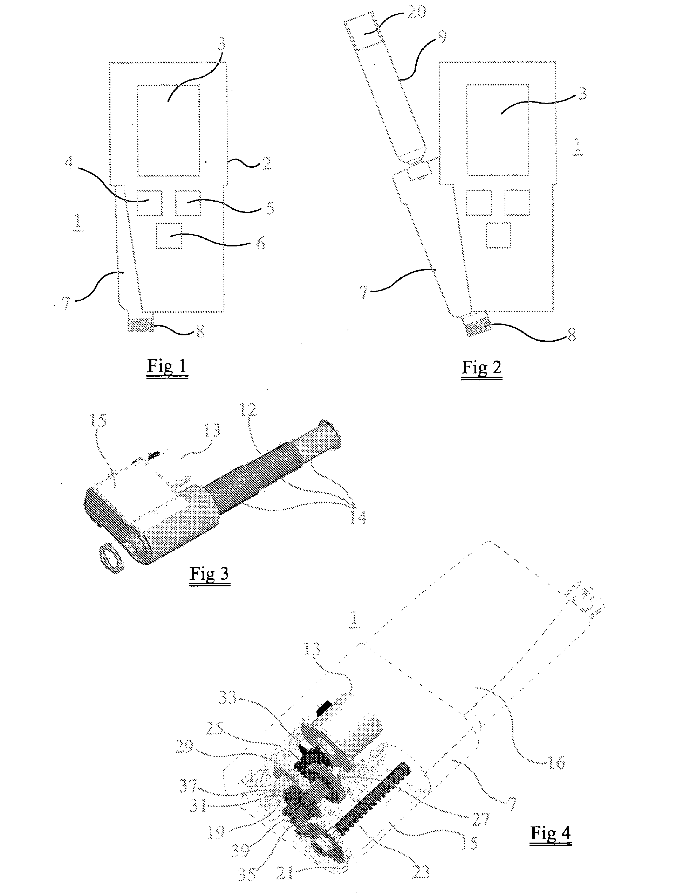

In figure 1, a medicament delivery device 1 comprises a case 2 having a display 3 for displaying functional information relating to the operation of the medicament delivery device, including the set dose, number of doses remaining in the medicament cartridge. User interface buttons 4, 5 and 6 are provided to allow the user to operate the injector including priming, setting a dose, opening a medicament cartridge holder and door 7, and activating the dispensing of the set dose. A threaded needle attachment 8 is provided to which a needle can be attached for dose delivery and subsequently removed and discarded. A cover (not shown) may be provided to fit over the lower portion of the case 2 to assist in protect the device from the ingress of particles and fluid. Figure 2 shows the medicament delivery device 1 with the cartridge holder and door 7 in an open position for receiving a replacement medicament cartridge 9.

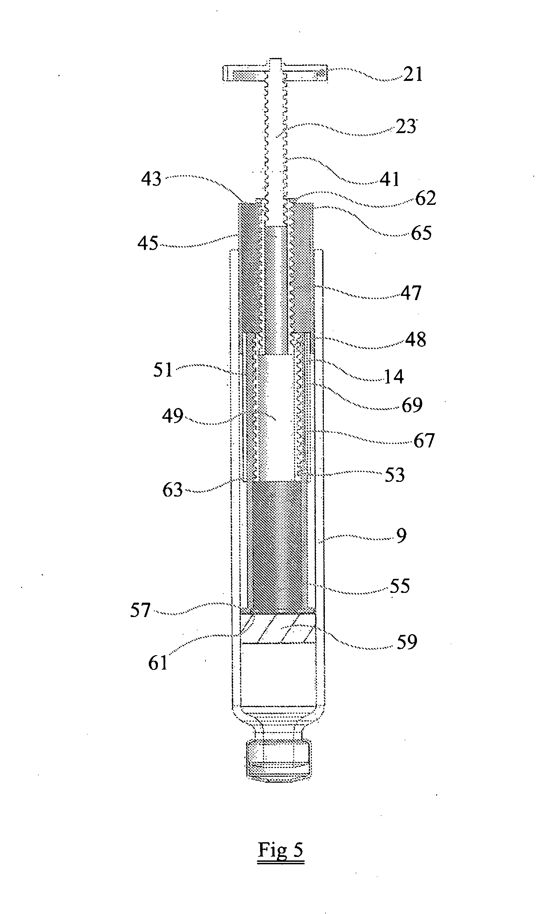

Figure 3 is a perspective view of a telescopic piston rod assembly 12, described in more detail below with reference to Figures 5-8, together with a motor 13, and a housing 15 which houses a drive mechanism for transferring drive to the telescopic piston rod assembly 12. The telescopic piston rod assembly 12 is provided with keys 14 which will be described below with reference to Figure 5. This assembly may be housed in a chassis 16 of the medicament delivery device 1. The chassis of the medicament delivery device 1 comprises space for a battery (not shown) and space for the medicament cartridge holder and door 7.

Figure 4 is a sectional perspective view of the assembly of Figure 3 with the housing removed 15. This view shows a drive mechanism 17 which includes a gear train 19, input gear 21 and input drive 23 of the telescopic piston rod assembly 12. The gear train 19 comprises a motor pinion 25 and three compound gear stages 27, 29 and 31. The motor pinion 25 is retained on a shaft of the motor 13 with an interference fit and has a pair of moulded flags 33 disposed at 180 degrees about the motor pinion 25. The flags 33 interrupt a motion detect optical sensor (not shown) in the chassis, the sensor being operative to provide an output corresponding to and indicative of the speed of rotation of the motor 13. The motor pinion 25, each compound gear stages 27 to 31 and the input gear 21 together serve to reduce the rotational speed of the drive between the motor 13 and input drive 23 in any suitable ratio which may be, for example, 62.8:1 so that for every revolution of the input drive 23, the motor rotates 62.8 times. A transfer gear 35, located between the compound gear 31 and the input gear 21 is non-reducing and so serves to provide a mechanical link. The compound reduction gear stages 27 to 31 are mounted on two parallel stainless steel pins 37 and 39. The input gear 21 is supported within the housing 15 so as to be rotatably engaged with the transfer gear 35 such as to drivingly rotate the input drive 23. The input gear 21 and input drive 23 are non-movable in an axial direction.

Figure 5 is a sectional view of the telescopic piston rod assembly 12 in an extended position within the medicament cartridge 9. The telescopic piston rod assembly 12 comprises an input drive 23 at the proximal end which is non-rotatably fixed the input gear 21. The input drive 23 has a threaded outer surface 41 which co-operates with an internally threaded portion 43 of a first cylinder 45 of an intermediate coupling, which also has a threaded outer surface 47. The threaded outer surface 47 of the first cylinder 45 co-operates with an internally threaded portion 48 at the proximal end of a second cylinder 49 of the intermediate coupling, the second cylinder also having a threaded outer surface 51. The distal end 63 of the threaded outer surface 51 co-operates with an internally threaded portion 53 at the proximal end of a plunger 55. The plunger 55 is provided with an outwardly extending flange 57 at its distal end for spreading a load exerted by the plunger 55 on a bung 59 of the medicament cartridge 9. The load exerted on the bung 59 arises when the motor 13 drives the telescopic piston rod assembly 12 such that it extends to drive the bung 55 along the medicament cartridge 9 in order to expel medicament therefrom during use. An inwardly extending annular insert 61 is provided at the distal end of the plunger 55 and serves to stop the plunger retracting beyond a distal end 63 of the second cylinder 49. A corresponding end stop 62 is mounted on the proximal end of the first cylinder 45 and serves to stop the second cylinder 49 from retracting beyond a proximal end of the first cylinder 45. The distal ends of the input drive 23, first cylinder 45 and the second cylinder 49 are provided with closed threads to prevent them from becoming de-coupled from one another.

The plunger 55 is keyed to the housing by means of a first sleeve 65 and a second sleeve 67. A key 14 (see Figure 3) is provided so as to render the plunger 55 and the first and second sleeves 65, 67 non-rotatable relative to one another and the housing. Key 14 is provided by a longitudinal (i.e. axially extending) groove or recess running along the outer surface of each of the plunger 55 and the first and second sleeves 65, 67. The groove or recess cooperates with a corresponding peg or spline 69 provided in the inner surface of the housing (not shown) as well as the inner surface of the first sleeve 65 and the second sleeve 67.

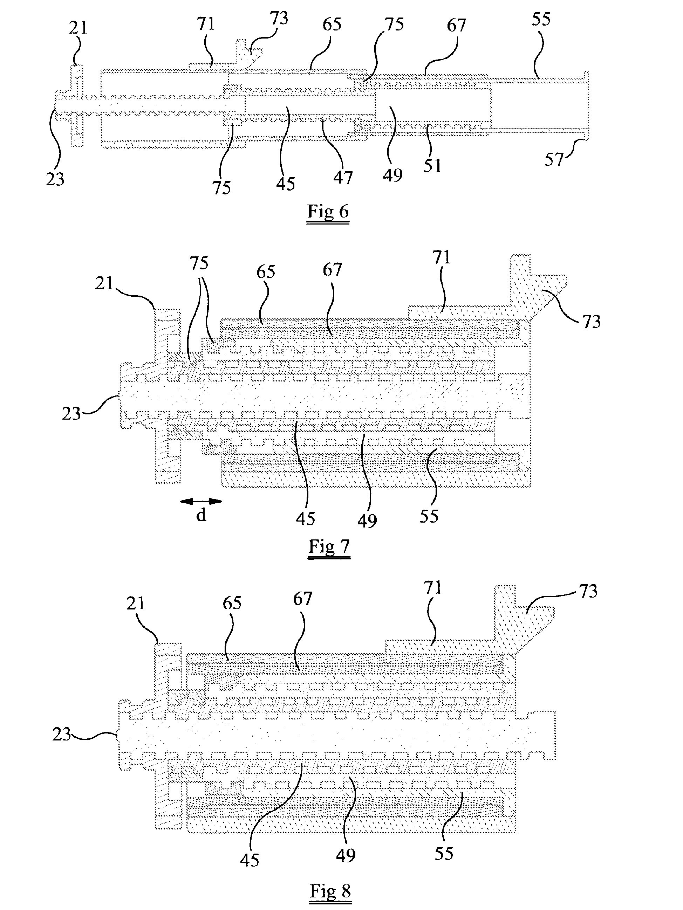

Figures 6 to 8 show a modified embodiment of the device according to the current invention. Figure 6 shows the telescopic piston rod assembly 12 in an extended position together with the detail of a locking member 71 which sits rotatably and axially movably within the housing 15. The locking member 71, which will be described in more detail below with reference to Figures 7 and 8, has an outwardly extending tab 73 which is operative for engaging/disengaging with a door catch release mechanism (not shown) depending on its axial position relative to the input gear 21 and input drive 23. Clip-on stop rings 75 are additionally provided on the proximal ends of each one of the first and second threaded cylinders 45, 49 for preventing the components from decoupling. The clip-on stop rings 75 are provided with sufficient stop strength on the one hand but without requiring a bonding process on the other.

Figures 7 and 8 are sectional views of the telescopic piston rod assembly 12 shown in a retracted state and with a door locking member in a forward 'door closed' position and rearward 'door release' position respectively. As the drive mechanism drives the input gear 21 and the input drive 23 to retract the telescopic piston rod assembly 12, the plunger 55, the first and second cylinders 47 and 49 eventually move into a retracted state as illustrated in Figure 7. In this position, there remains a gap d between the proximal end of the locking member 71 and the input gear 21. In this position, the tab 73 has not progressed or retracted far enough to disengage a medicament cartridge door opening latch (not shown). When it is desired to replace the medicament cartridge 9, a motor controller (not shown) drives the motor 13 so that the drive mechanism 17 drives the input drive 23 to retract the telescopic piston rod assembly 12 until the gap d is closed, hence disengaging the tab 73 and releasing the door opening latch.

The operation of the medicament delivery device 1 described above will now be described.

Extension of the telescopic piston rod assembly 12 from an initial retracted state (as shown in Figures 7 and 8) where the plunger 55, the first and second cylinders 45, 49 and the sleeves 65, 67 lie within one another is performed in three phases or stages.

In phase 1, the input drive 23 is rotated by the gear train 19 of the drive mechanism 17 engaging with the input gear 21. The input drive 23 therefore transfers axial forces into the housing 15 while transferring the torque input from the drive mechanism 17 into the first cylinder 45. During phase 1, the first cylinder 45, the second cylinder 49, the first and second sleeves 65, 67 and the plunger 55 move bodily and axially (but without rotation) along the length of the input drive 23. This occurs on account of: the first cylinder 45 transferring the torque input from the input drive 23 and providing an axial force into the second cylinder 49; the second cylinder 49 transferring the axial input force from the first cylinder 45 and transferring the axial input force into the plunger 55; the first sleeve 65 reacts the torque from the second sleeve 67 and transfers it into the housing 15; the second sleeve 67 reacts the torque from the plunger 55 and transfers it into the first sleeve 65; and the plunger 55 transfers the axial force from the plunger and transfers it into the medicament cartridge 9.

Phase 2 begins when the first cylinder 45 has fully extended, that is, has moved down the length of the input drive 23 until relative rotation between them is arrested by an extension stop 75 or closed thread. At this point, the first cylinder 45 has reached its axial limit and no longer moves axially but rotates with the input drive 23. The first cylinder 45 then transfers the input torque from the input drive 23 into the second cylinder 49, thus causing the second cylinder to progress axially along the first cylinder without rotation. The second cylinder 49 transfers the torque input from the first cylinder 45 and provides an axial force into the plunger 55. The plunger moves axially without rotation. The plunger 55 transfers the torque input from the second cylinder 67 and provides an axial force into the medicament cartridge 9.

During phase 2, the first sleeve 65 does not move but reacts the torque from the second sleeve 67 and transfers it into the drive mechanism housing 15. The second sleeve 67 does not move and reacts the torque from the plunger 55 and transfers it into the first sleeve 65 so that it does not rotate.

Phase 3 begins when the second cylinder 49 has fully extended, that is, has moved down the length of the first cylinder 45 until relative rotation is arrested by a second extension stop 75 or closed thread, it no longer moves axially, as it has reached its axial limit, but rotates with the input drive 23 and the first cylinder 45. The second cylinder 49 then transfers the torque from the first cylinder 45 into the plunger 55 so that the plunger moves axially along the length of the second cylinder without rotating. The plunger 55 is keyed to the second sleeve 67 so that it is prevented from rotating with the input drive 23, thereby achieving relative rotation with the second cylinder 49. A third extension stop in the form of a closed thread is provided to arrest relative rotation between the plunger 55 and the second cylinder 67 when the plunger is fully extended relative thereto.

The medicament delivery device 1 includes an electronic control system (not shown) for controlling the operation of the device. The control system is operative for controlling the motor 13 to drive the input drive 23 via the drive mechanism 17. Sensors (not shown) are provided for sensing the state of the telescopic piston rod assembly 12 and providing the user with operational information.

## Claims

1. A medicament delivery device (1) comprising: a housing (2), a telescopic piston rod assembly (12) for driving a bung (20) of a medicament container (9); and a drive mechanism (17) for the telescopic piston rod assembly,
wherein the telescopic piston rod assembly (12) has an input drive (23) telescopically coupled to a plunger (55) for driving the bung (20) via an intermediate coupling;
wherein the intermediate coupling comprises: first and second members (45, 49) telescopically coupled to one another, the plunger (55) being telescopically coupled to one of the first and second members (45, 49) and the input drive (23) being telescopically coupled to the other one of the first and second members (45, 49); and a key (14) for restraining rotation of the plunger (55) relative to the housing (2);
whereby as the drive mechanism (17) drives the input drive (23), the plunger (55), first and second members (45, 49) and the input drive (23) telescopically expand or retract with respect to one another.

2. A medicament delivery device according to claim 1, wherein the key (14) comprises first and second sleeves (65, 67) that are telescopically coupled and keyed to one another, the plunger (55) and the housing (2) so that they are axially but not rotationally moveable relative to the housing (2).

3. A medicament delivery device according to claim 1 or claim 2, wherein the telescopic relationship between the plunger (55), the first and second members (45, 49), and the input drive (23) are such that their respective diameters progressively decrease from the plunger (55) to the input drive (23).

4. A medicament delivery device according to claim 3, wherein the telescopic relationship between the plunger (55), the first and second sleeves (65, 67) is such that their respective diameters progressively increase from the plunger (55) to the input drive (23).

5. A medicament delivery device according to any one of the preceding claims, wherein an input gear (21) is provided at the end of the input drive (23) remote from the first member (45) for engagement with a gear train (19) of the drive mechanism (17).

6. A medicament delivery device according to any one of the preceding claims wherein extension of the telescopic piston rod assembly (12) from an initial retracted state where the plunger (55), the first and second members (45, 49) and the sleeves (65, 67) lie at least substantially within one another is performed progressively.

7. A medicament delivery device according to claim 6, wherein the input drive (23) is rotated to move axially so that the telescopic piston rod assembly (12) moves bodily and axially but without rotation along the length of the input drive.

8. A medicament delivery device according to claim 7, wherein the first member (45) reaches an axial limit so as to rotate with the input drive (23) to transfer the input torque from the input drive (23) into the second member (49), thus causing the second member (49) and the plunger (55) to progress axially along the first member (45) without rotation.

9. A medicament delivery device according to claim 8, wherein the second member (49) reaches an axial limit so as to rotate with the input drive (23) and the first member (45) thus transferring the torque from the first member (45) into the plunger so that the plunger (55) moves axially along the length of the second member (49) without rotating.

10. A medicament delivery device according to any one of the preceding claims, wherein the first and second sleeves (65, 67) each have a length that is substantially equal to the length of each of the first and second members (45, 49) so as to minimise the length of the telescopic piston rod assembly (12) when in a retracted state.

11. A medicament delivery device according to any previous claim, wherein the housing (2) includes a receptacle (7) for a user replaceable medicament cartridge (9).

12. An auto-injector including a medicament delivery device according to any one of the preceding claims

## Patentansprüche

1. Vorrichtung zur Medikamentenverabreichung (1), umfassend: ein Gehäuse (2), eine teleskopische Kolbenstangenanordnung (12) zum Antreiben eines Stopfens (20) eines Arzneimittelbehälters (9), und einen Antriebsmechanismus (17) für die teleskopische Kolbenstangenanordnung,
worin die teleskopische Kolbenstangenanordnung (12) einen Eingangsantrieb (23) aufweist, der zum Antreiben des Stopfens (20) über eine Zwischenkupplung teleskopisch mit einem Kolben (55) verbunden ist;
worin die Zwischenkupplung Folgendes umfasst: erste und zweite Elemente (45, 49), die teleskopisch miteinander verbunden sind, wobei der Kolben (55) teleskopisch mit einem der ersten und zweiten Elemente (45, 49) verbunden ist und der Eingangsantrieb (23) teleskopisch mit den anderen der ersten und zweiten Elemente (45, 49) verbunden ist; und einen Keil (14) zur Begrenzung der Drehung des Kolbens (55) im Verhältnis zu dem Gehäuse (2);
wobei sich der Kolben (55), die ersten und zweiten Elemente (45, 49) und der Eingangsantrieb (23) mit Bezug aufeinander teleskopisch ausziehen oder zurückziehen, wenn der Antriebsmechanismus (17) den Eingangsantrieb (23) antreibt.

2. Vorrichtung zur Medikamentenverabreichung nach Anspruch 1, worin der Keil (14) erste und zweite Hülsen (65, 67) umfasst, die teleskopisch miteinander, mit dem Kolben (55) und dem Gehäuse (2) verbunden und verkeilt sind, so dass sie bezüglich des Gehäuses (2) axial beweglich, aber nicht drehend beweglich sind.

3. Vorrichtung zur Medikamentenverabreichung nach Anspruch 1 oder Anspruch 2, worin die teleskopische Beziehung zwischen dem Kolben (55), den ersten und zweiten Elementen (45, 49) und dem Eingangsantrieb (23) so ist, dass ihre jeweiligen Durchmesser von dem Kolben (55) zu dem Eingangsantrieb (23) progressiv abnehmen.

4. Vorrichtung zur Medikamentenverabreichung nach Anspruch 3, worin die teleskopische Beziehung zwischen dem Kolben (55)und den ersten und zweiten Hülsen (65, 67) so ist, dass ihre jeweiligen Durchmesser von dem Kolben (55) zu dem Eingangsantrieb (23) progressiv zunehmen.

5. Vorrichtung zur Medikamentenverabreichung nach einem der vorhergehenden Ansprüche, worin ein Eingangsgetriebe (21) am Ende des Eingangsantriebs (23), entfernt von dem ersten Element (45), zum Eingriff mit einem Getriebezug (19) des Antriebsmechanismus (17) bereitgestellt ist.

6. Vorrichtung zur Medikamentenverabreichung nach einem der vorhergehenden Ansprüche, worin das Ausziehen der teleskopischen Kolbenstangenanordnung (12) aus einem anfänglich zurückgezogenen Zustand, in dem der Kolben (55), die ersten und zweiten Elemente (45, 49) und die Hülsen (65, 67) zumindest teilweise ineinander liegen, progressiv erfolgt.

7. Vorrichtung zur Medikamentenverabreichung nach Anspruch 6, worin der Eingangsantrieb (23) gedreht wird, damit er sich axial bewegt, so dass sich die teleskopische Kolbenstangenanordnung (12) körperlich und axial aber ohne Drehung über die Länge des Eingangsantriebs bewegt.

8. Vorrichtung zur Medikamentenverabreichung nach Anspruch 7, worin das erste Element (45) eine axiale Grenze erreicht, um sich mit dem Eingangsantrieb (23) zu drehen und das Eingangsdrehmoment von dem Eingangsantrieb (23) in das zweite Element (49) zu übertragen, wodurch sich das zweite Element (49) und der Kolben (55) axial entlang des ersten Elements (45) ohne Drehung fortbewegen.

9. Vorrichtung zur Medikamentenverabreichung nach Anspruch 8, worin das zweite Element (49) eine axiale Grenze erreicht, um sich mit dem Eingangsantrieb (23) und dem ersten Element (45) zu drehen, wodurch das Drehmoment von dem ersten Element (45) in den Kolben übertragen wird, so dass sich der Kolben (55) axial über die Länge des zweiten Elements (49) ohne Drehung bewegt.

10. Vorrichtung zur Medikamentenverabreichung nach einem der vorhergehenden Ansprüche, worin die ersten und zweiten Hülsen (65, 67) jeweils eine Länge aufweisen, die im Wesentlichen der Länge jedes der ersten und zweiten Elemente (45, 49) entspricht, um die Länge der teleskopischen Kolbenstangenanordnung (12) in einem zurückgezogenen Zustand zu minimieren.

11. Vorrichtung zur Medikamentenverabreichung nach einem der vorhergehenden Ansprüche, worin das Gehäuse (2) eine Aufnahme (7) für eine von einem Anwender auswechselbare Arzneimittelkassette (9) aufweist.

12. Autoinjektor mit einer Vorrichtung zur Medikamentenverabreichung nach einem der vorhergehenden Ansprüche.

## Revendications

1. Dispositif d'administration de médicaments (1), comprenant : un boîtier (2), un ensemble de tige de piston télescopique (12) pour entraîner un bouchon (20) d'un contenant de médicaments (9) ;
et un mécanisme d'entraînement (17) pour l'ensemble de tige de piston télescopique, l'ensemble de tige de piston télescopique (12) ayant un entraînement d'entrée (23) accouplé de manière télescopique à un plongeur (55) pour entraîner le bouchon (20) par le biais d'un accouplement intermédiaire ;
l'accouplement intermédiaire comprenant : des premier et deuxième organes (45, 49) accouplés de manière télescopique l'un à l'autre, le plongeur (55) étant accouplé de manière télescopique à l'un des premier et deuxième organes (45, 49) et l'entraînement d'entrée (23) étant accouplé de manière télescopique à l'autre des premier et deuxième organes (45, 49) ; et une clavette (14) pour limiter la rotation du plongeur (55) par rapport au boîtier (2) ;
dans lequel, à mesure que le mécanisme d'entraînement (17) entraîne l'entraînement d'entrée (23), le plongeur (55), les premier et deuxième organes (45, 49) et l'entraînement d'entrée (23) se déploient ou se rétractent de manière télescopique les uns par rapport aux autres.

2. Dispositif d'administration de médicaments selon la revendication 1, dans lequel la clavette (14) comprend des premier et deuxième manchons (65, 67) qui sont accouplés de manière télescopique et clavetés l'un à l'autre, au plongeur (55) et au boîtier (2), de telle sorte qu'ils soient déplaçables axialement mais pas en rotation par rapport au boîtier (2).

3. Dispositif d'administration de médicaments selon la revendication 1 ou la revendication 2, dans lequel la relation télescopique entre le plongeur (55), les premier et deuxième organes (45, 49), et l'entraînement d'entrée (23) est telle que leurs diamètres respectifs diminuent progressivement depuis le plongeur (55) jusqu'à l'entraînement d'entrée (23).

4. Dispositif d'administration de médicaments selon la revendication 3, dans lequel la relation télescopique entre le plongeur (55) et les premier et deuxième manchons (65, 67) est telle que leurs diamètres respectifs augmentent progressivement depuis le plongeur (55) jusqu'à l'entraînement d'entrée (23).

5. Dispositif d'administration de médicaments selon l'une quelconque des revendications précédentes, dans lequel un engrenage d'entrée (21) est prévu à l'extrémité de l'entraînement d'entrée (23) éloignée du premier organe (45) en vue de l'engagement avec un train d'engrenages (19) du mécanisme d'entraînement (17).

6. Dispositif d'administration de médicaments selon l'une quelconque des revendications précédentes, dans lequel l'extension de l'ensemble de tige de piston télescopique (12) depuis un état rétracté initial dans lequel le plongeur (55), les premier et deuxième organes (45, 49) et les manchons (65, 67) sont situés au moins substantiellement les uns dans les autres, s'effectue progressivement.

7. Dispositif d'administration de médicaments selon la revendication 6, dans lequel l'entraînement d'entrée (23) est tourné de manière à se déplacer axialement de telle sorte que l'ensemble de tige de piston télescopique (12) se déplace physiquement et axialement mais sans rotation le long de la longueur de l'entraînement d'entrée.

8. Dispositif d'administration de médicaments selon la revendication 7, dans lequel le premier organe (45) atteint une limite axiale de manière à tourner avec l'entraînement d'entrée (23) pour transférer le couple d'entrée depuis l'entraînement d'entrée (23) jusque dans le deuxième organe (49), pour ainsi amener le deuxième organe (49) et le plongeur (55) à progresser axialement le long du premier organe (45) sans rotation.

9. Dispositif d'administration de médicaments selon la revendication 8, dans lequel le deuxième organe (49) atteint une limite axiale de manière à tourner avec l'entraînement d'entrée (23) et le premier organe (45), transférant ainsi le couple depuis le premier organe (45) jusque dans le plongeur de manière à ce que le plongeur (55) se déplace axialement le long de la longueur du deuxième organe (49) sans tourner.

10. Dispositif d'administration de médicaments selon l'une quelconque des revendications précédentes, dans lequel le premier et le deuxième manchon (65, 67) présentent chacun une longueur qui est substantiellement égale à la longueur de chacun des premier et deuxième organes (45, 49) de manière à réduire à un minimum la longueur de l'ensemble de tige de piston télescopique (12) lorsqu'il est dans un état rétracté.

11. Dispositif d'administration de médicaments selon l'une quelconque des revendications précédentes, dans lequel le boîtier (2) comporte un récipient (7) pour une cartouche de médicaments (9) remplaçable par un utilisateur.

12. Auto-injecteur comportant un dispositif d'administration de médicaments selon l'une quelconque des revendications précédentes.
